# EUROPEAN PATENT SPECIFICATION

(11) **EP 0 115 771 B1**
(45) Date of publication and mention of the grant of the patent: **01.03.1995**
(21) Application number: 84100080.5
(22) Date of filing: 05.01.1984
(51) Int. Cl.: C08G 73/02, C08G 81/00, C08G 69/44, C08G 63/68, C08F 283/00

(54) **Dense star polymers and a process for producing dense star polymers**
Sternförmige Polymere mit hoher Dichte an Endgruppen und Verfahren zur Herstellung solcher Polymere
Polymères en étoile ayant des groupes terminaux à grande densité et procédé de préparation de ces polymères

(30) Priority: 07.01.1983 US 456226
(43) Date of publication of application: 15.08.1984
(60) Divisional application: 94102257.6
(73) Proprietor: THE DOW CHEMICAL COMPANY, Midland Michigan 48640-1967 (US)
(72) Inventor: Tomalia, Donald A., Midland Michigan 48640 (US); Dewald, James R., Bay City Michigan 48706 (US)
(74) Representative: Weickmann, Heinrich, Dipl.-Ing.

(56) References cited:
- EP-A- 0 066 366
- DE-A- 2 529 065
- DE-A- 2 820 211
- US-A- 3 773 739
- US-A- 4 102 827
- US-A- 4 248 984
- US-A- 4 289 872

## Description

This invention relates to a novel class of branched polymers containing dendritic branches having functional groups uniformly distributed on the periphery of such branches. This invention also relates to processes for preparing such polymers as well as applications therefore.

Organic polymers are generally classified in a structural sense as either linear or branched. In the case of linear polymers, the repeating units (often called mers) are divalent and are connected one to another in a linear sequence. In the case of branched polymers, at least some of the mers possess a valency greater than 2 such that the mers are connected in a nonlinear sequence. The term "branching" usually implies that the individual molecular units of the branches are discrete from the polymer backbone, yet have the same chemical constitution as the polymer backbone. Thus, regularly repeating side groups which are inherent in the monomer structure and/or are of different chemical constitution than the polymer backbone are not considered as branches, e.g., dependent methyl groups of linear polypropylene. To produce a branched polymer, it is necessary to employ an initiator, a monomer, or both that possess at least three moieties that function in the polymerization reaction. Such monomer or initiators are often called polyfunctional. The simplest branched polymers are the chain branched polymers wherein a linear backbone bears one or more essentially linear pendant groups. This simple form of branching, often called comb branching, may be regular wherein the branches are uniformly and regularly distributed on the polymer backbone or irregular wherein the branches are distributed in nonuniform or random fashion on the polymer backbone. See T. A. Orofino, Polymer, 2, 295-314 (1961). An example of regular comb branching is a comb branched polystyrene as described by T. Altores et al. in J. Polymer Sci., Part A, Vol. 3, 4131-4151 (1965) and an example of irregular comb branching is illustrated by graft copolymers as described by Sorenson et al. in "Preparative Methods of Polymer Chemistry", 2nd Ed., Interscience Publishers, 213-214 (1968).

Another type of branching is exemplified by cross-linked or network polymers wherein the polymer chains are connected via tetravalent compounds, e.g., polystyrene molecules bridged or cross-linked with diyinylbenzene. In this type of branching, many of the individual branches are not linear in that each branch may itself contain groups pendant from a linear chain. More importantly in network branching, each polymer macromolecule (backbone) is cross-linked at two or more sites to two other polymer macromolecules. Also the chemical constitution of the cross-linkages may vary from that of the polymer macromolecules. In this so-called cross-linked or network branched polymer, the various branches or cross-linkages may be structurally similar (called regular cross-linked) or they may be structurally dissimilar (called irregularly cross-linked). An example of regular cross-linked polymers is a ladder-type poly(phenylsilsesquinone) as described by Sorenson et al., supra, at page 390. The foregoing and other types of branched polymers are described by H. G. Elias in Macromolecules, Vol. I. Plenum Press, New York (1977)

More recently, there have been developed polymers having so-called star structured branching wherein the individual branches radiate out from a nucleus and there are at least 3 branches per nucleus. Such star branched polymers are illustrated by the poly-quaternary compositions described in USP Nos. 4,036,808 and 4,102,827. Star branched polymers prepared from olefins and unsaturated acids are described in USP 4,141,847. The star branched polymers offer several advantages over polymers having other types of branching. For example, it is found that the star branched polymers may exhibit higher concentrations of functional groups thus making them more active for their intended purpose. In addition, such star branched polymers are often less sensitive to degradation by shearing which is a very useful property in formulations such as paints, in enhanced oil recovery and other viscosity applications. Additionally, the star branched polymers have relatively low intrinsic viscosities even at high molecular weight.

While the star branched polymers offer many of the aforementioned advantages over polymers having more conventional branching, it is highly desirable to provide polymers which exhibit even greater concentrations of functional groups per unit volume of the polymer macromolecule as well as a more uniform distribution of such functional groups in the exterior regions of the macromolecule. In addition, it is often desirable to provide polymers having macromolecular configurations that are more spheroidal and compact than are the star branched polymers.

In its broadest aspect, this invention is a dense star polymer having at least three symmetrical core branches emanating from a core, each core branch having at least one terminal group provided that (1) the ratio of terminal groups to the core branches is greater than 2:1, (2) the density of terminal groups per unit volume in the polymer is at least 1.5 times that of a conventional star polymer having the same core, monomeric components, molecular weight and number of core branches, said monomeric components being such that each of the branches bears only one terminal group, and (3) the molecular volume is not more than 60 percent of the molecular volume of said conventional star polymer. The molecular volume is determined by dimensional studies using scaled Corey-Pauling molecular models. For purposes of this invention, the term "dense" as it modifies "star polymer" means that it has a smaller molecular volume than a conventional star polymer having the same molecular weight. While the number of terminal groups is greater for the dense star polymer molecule than in the conventional star polymer molecule, the chemical structure of the terminal groups is the same.

In a somewhat more limited and preferred aspect, this invention is a polymer having a novel ordered star branched structure (herein called starburst structure). Hereinafter this polymer having a starburst structure is called a dendrimer. Thus, a "dendrimer" is a polymer having a polyvalent core that is covalently bonded to at least three ordered, symmetrical dendritic (tree-like) branches which extend through at least two generations. As an illustration, an ordered second generation dendritic branch is depicted by the following configuration: 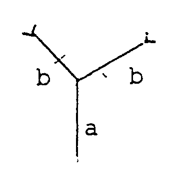
wherein "a" represents the first generation and "b" represents the second generation. An ordered, third generation dendritic branch is depicted by the following configuration: 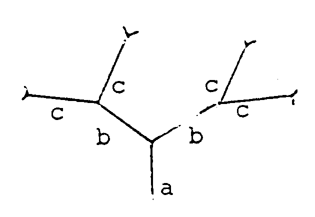
wherein "a" and "b" represent the first and second generation, respectively, and "c" represents the third generation. A primary characteristic of the ordered dendritic branch which distinguishes it from conventional branches of conventional polymers is the uniform or essentially symmetrical character of the branches as is shown in the foregoing illustrations. In addition, with each new generation, the number of terminal groups on the dendritic branch is an exact multiple of the number of terminal groups in the previous generation.

Another aspect of this invention is a process for producing the symmetrical dense star polymer comprising the steps of:
(i) contacting
   (1) a core compound having at least three nucleophilic or three electrophilic reactive atoms or groups (A₁-groups) with
   (2) an excess of a first organic coreactant having
      (a) one core reactive atom or group B₁ which is reactive with the A₁-groups and
      (b) at least two reactive atoms or groups A₂ which do not react with the A₁-group or the B₁-group under conditions sufficient to form a core adduct wherein each A₁-group has reacted with the core reactive group of a different molecule of the first coreactant;
(ii) contacting
   (1) the core adduct having at least twice the number of reactive groups as the core compound with
   (2) an excess of a second organic coreactant having
      (a) one adduct reactive atom or group B₂ which will react with the A₂-groups of the core adduct and
      (b) at least one reactive atom or group A₃ which does not react with the A₂-groups or the B₂-group under conditions sufficient to form a first generation adduct having a number of reactive groups that are at least twice the number of A₁-groups;
(iii) contacting
   the first generation adduct of step (ii) with an excess of a third organic coreactant having
   (a) one reactive atom or group B₃ that is reactive with the A₃-groups of the first generation adduct and
   (b) at least one reactive atom or group A₄ that does not react with the A₃-groups or the B₃-groups of the first generation adduct under conditions sufficient to form a second generation adduct,
   wherein the first coreactant differs from the second coreactant, and the second coreactant differs from the third coreactant, but the first and third coreactants may be the same or different compounds,
   and optionally repeating steps (ii) and (iii) the desired number of times and
   wherein each excess is 2:1 to 120:1 on a molar basis.

The dense star polymer of the invention is useful in such applications as demulsifiers for oil/water emulsions, wet strength agents in the manufacture of paper, agents for modifying viscosity in aqueous formulations such as paints and the like. For example, in a demulsification method, an emulsion of oil and water is contacted with a demulsifying amount of the dense star polymer under conditions sufficient to cause phase separation.

The dense star polymers of the present invention exhibit the following properties which are unique or are superior to similar properties of conventional star branched polymers and other branched polymers having similar molecular weight and terminal groups:
(a) greater branch density;
(b) greater terminal group density;
(c) greater accessibility of terminal groups to chemically reactive species; and
(d) lower viscosity.

In the dense star polymers of the present invention, the core is covalently bonded to at least three core branches with each core branch having a calculated length of at least 3 Angstrom units (A) (0.3 nm), preferably at least 4 A (0.4 nm), most preferably at least 6 A (0.6 nm). These polymers have an average of at least 3 and most preferably at least 4 terminal groups per polymer molecule. Preferably, the core branches have a dendritic character, most preferably an ordered dendritic character as defined hereinafter. In preferred dense star polymers, the terminal groups are functional groups that are sufficiently reactive to undergo addition or substitution reactions. Examples of such functional groups include amino, hydroxy, mercapto, carboxy, alkenyl, allyl, vinyl, amido, halo, urea, oxiranyl, aziridinyl, oxazolinyl, imidazolinyl, sulfonato, phosphonato, isocyanato and isothiocyanato. The dense star polymers differ from conventional star or star-branched polymers in that the dense star polymers have a greater concentration of terminal groups per unit of molecular volume than do conventional star polymers having an equivalent number of core branches and an equivalent core branch length. Thus, the density of terminal groups per unit volume in the dense star polymer is at least 1.5 times the density of terminal groups in the conventional star polymer, preferably at least 5 times, more preferably at least 10 times, most preferably from 15 to 50 times. The ratio of terminal groups per core branch in the dense polymer is greater than 2:1, preferably at least 3, most preferably from 4 to 1024. Preferably, for a given polymer molecular weight, the molecular volume of the dense star polymer is no more than 50 volume percent, more preferably from 16 to 50, most preferably from 7 to 40 volume percent of the molecular volume of the conventional star polymer.

In the preferred polyamidoamine dense star polymers, the density of terminal (primary) amine moieties in the polymer is readily expressed as the molar ratio of primary amine groups to the total of secondary and tertiary amine groups. In such polymers this 1° amine:(2°amino + 3° amine) is preferably from 0.37:1 to 1.33:1, more preferably from 0.69:1 to 1.2:1, most preferably from 1.1:1 to 1.2:1.

The dendrimers of the present invention are characterized as having a polyvalent core that is covalently bonded to at least three ordered, symmetrical dendritic branches which extend through at least two generations. Such ordered branching can be illustrated by the following sequence wherein G indicates the number of generations: 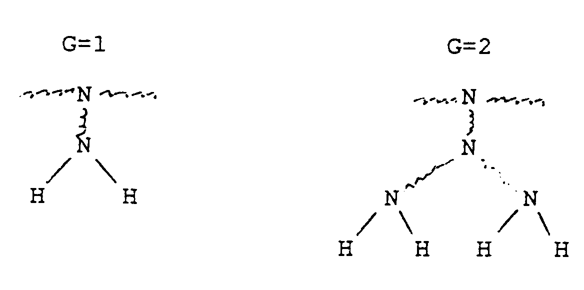 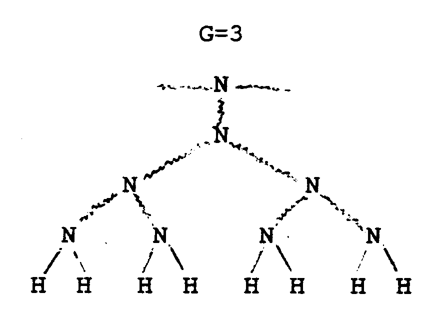
Mathematically, the relationship between the number of terminal groups on a dendritic branch and the number of generations of the branch can be represented as follows: 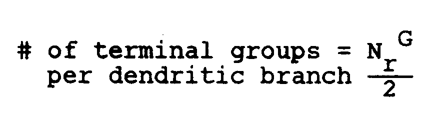
wherein G is the number of generations and Nᵣ is the repeating unit multiplicity which is at least 2 as in the case of amines. The total number of terminal groups in the dendrimer is determined by the following: 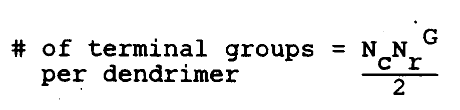
wherein G and Nᵣ are as defined before and N_{c} represents the valency (often called core funtionality) of the core compound. Accordingly, the dendrimers of the present invention can be represented in its component parts as follows: 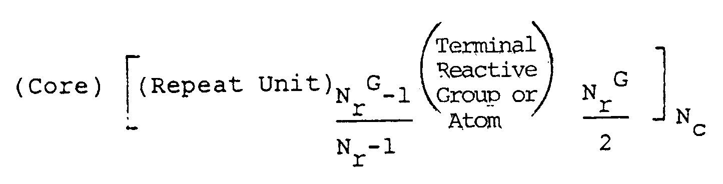
wherein the Core, Terminal Reactive Group or Atom, G, and N_{c} are as defined before and the Repeat Unit has a valency or functionality of Nᵣ + 1 wherein Nᵣ is as defined before.

An illustration of a functionally active dendrimer of a ternary or trivalent core which has three ordered, second generation dendritic branches is depicted by the following configuration: 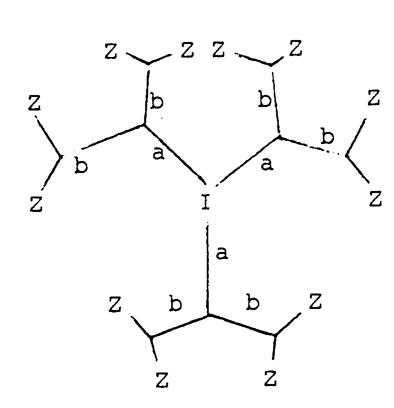
wherein I is a trivalent core atom or molecule having a covalent bond with each of the three dendritic branches, Z is a terminal reactive group or atom and "a" and "b" are as defined hereinbefore. An example of such a ternary dendrimer is polyamidoamine represented by the following structural formula: 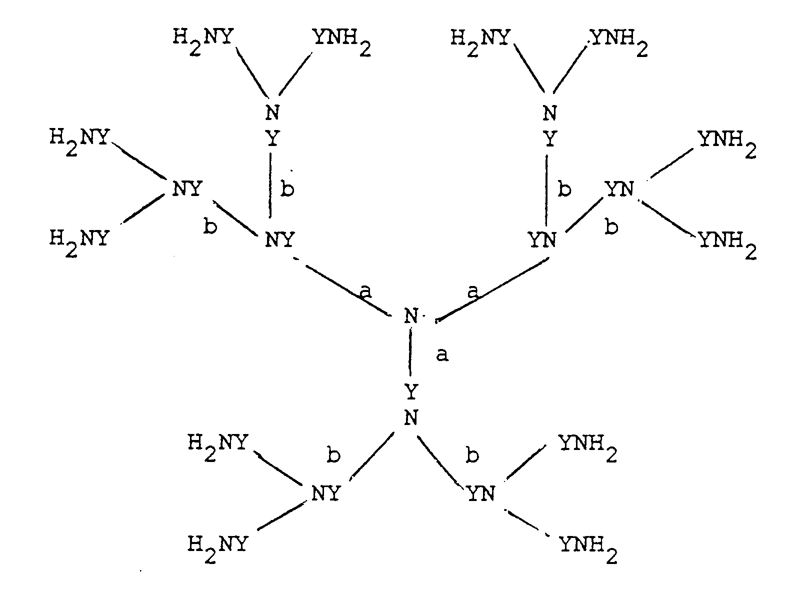
wherein Y represents a divalent amide group or atom such as 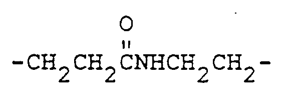
and "a" and "b" indicate first and second generations, respectively. In these two illustrations, N_{c} is 3 and Nᵣ is 2. In the latter of the two illustrations, the Repeat Unit is YN. While the foregoing configuration and formula illustrate a trivalent core, the core atom or molecule may be any polyvalent or polyfunctional group or atom, preferably a polyvalent or polyfunctional group or atom having from 2 to 2300 valence bonds or functional sites available for bonding with the dendritic branches, most preferably from 2 to 200 valence bonds or functional sites.

Accordingly, this dense star must have at least 2 generations in order to exhibit the desired density of terminal groups.

Also, Y may be any other divalent organic group or atom such as, for example, alkylene, alkylene oxide, alkyleneamine, with the depicted amide groups being the most preferred. In addition to amine, the terminal groups of the dendrimer may be any functionally active group or atom that can be used to propagate the dendritic branch to the next generation. Examples of such other groups include carboxy, aziridinyl, oxazolinyl, haloalkyl, oxiranyl, hydroxy and isocyanato, with amine or carboxylic ester groups being preferred.

While the dendrimers preferably have dendritic branches having 2 to 6 generations, dendrimers having dendritic branches up to 12 generations are suitably made and employed in the practice of this invention.

More preferably, the amidoamine dendimers of this invention are represented by the formula: 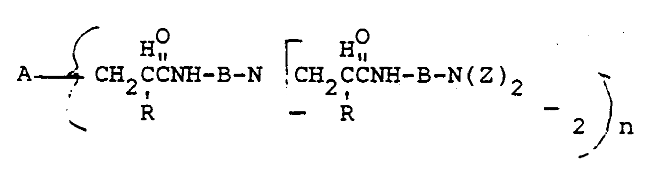
wherein A is a n-valent core derived from a nucleophilic compound, R is hydrogen or lower alkyl, B is a divalent group or atom capable of linking amine groups, n is an integer of 3 or more corresponding to the number of the core branches and Z is hydrogen or 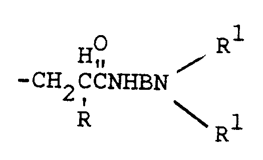
wherein R¹ is hydrogen or 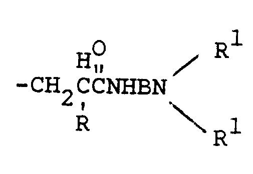
wherein each generation is represented by 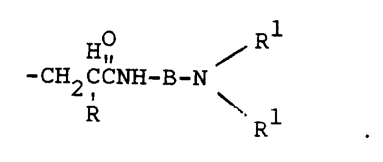
More preferably A is a core such as 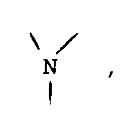 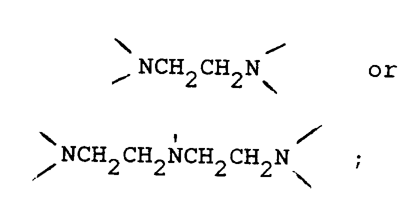
R is hydrogen or methyl; B is the divalent residue of a polyamine, most preferably an alkylene polyamine such as ethylene diamine or a polyalkylene polyamine such as triethylene tetramine; n is an integer from 3 to 2000, more preferably from 3 to 1000, most preferably from 3 to 125, and Z is most preferably

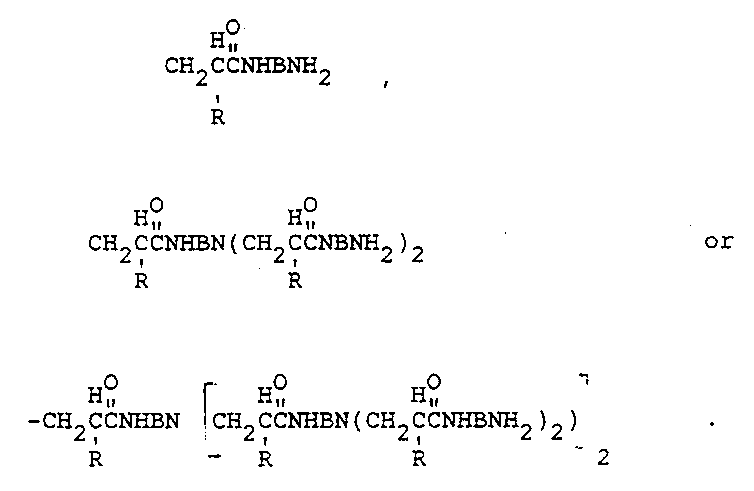

The dense star polymers of this invention are readily prepared by reacting a compound capable of generating a polyvalent core with a compound or compounds which causes propagation of dendritic branches from the core. In one method of preparing these dendrimers (herein called the successive excess reactant method), it is essential to maintain an excess of coreactant to reactive moieties in the terminal groups in the core, core adduct or subsequent adducts and dendrimers in order to prevent cross-linking and to maintain the ordered character of the dendritic branches. In general, this excess of coreactant to reactive groups or atoms in the terminal groups is from 1:1 to 120:1, preferably from 3:1 to 20:1 on a molar basis.

Alternatively, the compound capable of generating a polyvalent core, W(X)ₙ, wherein W is the polyvalent core atom and is covalently bonded to nX reactive terminal groups (n≧2), is reacted with a partially protected multi functional reagent, T(̵U)Ⓥₘ, wherein U represents a multivalent group or atom covalently bonded to mⓋ protected groups or atoms (m≧2), and to one T, a group or atom capable of reacting with X to form W[(X'-T')̵UⓋₘ]ₙ, wherein X' and T' represent the residue of reaction between X and T. This first generation compound is then subjected to activation conditions whereby the Ⓥ groups or atoms are made reactive (deprotected) and reacted with the partially protected multifunctional reagent, T-U-Ⓥₘ, to form the second generation protected dendrimer, W⁅{(X'-T')̵UV}ₘT'-UⓋₘ]ₙ. This protected dendrimer can be activated and reacted again in a similar manner to provide the third generation protected dendrimer. Both the successive excess reactant and the partially protected reactant method are specifically illustrated hereinafter.

The successive excess reactant method of preparing the dendrimers is illustrated by the preparation of the aforementioned ternary dendritic polyamidoamine. In this method, ammonia, a nucleophilic core compound, is first reacted with methyl acrylate under conditions sufficient to cause the Michael addition of one molecule of the ammonia to three molecules of the methyl acrylate to form the following core adduct: 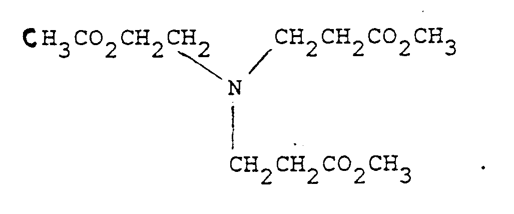
Following removal of unreacted methyl acrylate, this compound is then reacted with excess ethylenediamine under conditions such that one amine group of the ethylenediamine molecule reacts with the methyl carboxylate groups of the core adduct to form a first generation adduct having three amidoamine groups represented by the formula: 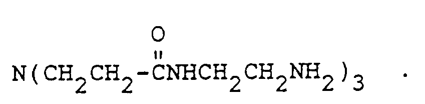
The molar excess of ethylene diamine to methyl acrylate groups is preferably from 4:1 to 50:1. Following removal of unreacted ethylenediamine, this first generation adduct is then reacted with excess methyl acrylate under Michael's addition conditions to form a second generation adduct having terminal methyl ester groups 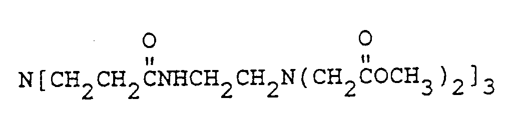
which is then reacted with excess ethylenediamine under amide forming conditions to produce the desired polyamidoamine dendrimer having ordered, second generation dendritic branches with terminal amine groups The molar excess of coreactant to reactive groups or atoms in each case is preferably from 1.1:1 to 120:1, most preferably from 3:1 to 10:1. Similar dendrimers containing amidoamine groups can be made by using organic amines as the core compound, e.g., ethylenediamine which produces a tetra-branched dendrimer or diethylenetriamine which produces a penta-branched dendrimer.

Other dendrimers made by the successive excess reactant method are polysulfides made by (1) reacting a polythiol, C(CH₂SH)₄, under basic conditions with epichlorosulfide to form the first generation polyepisulfide, 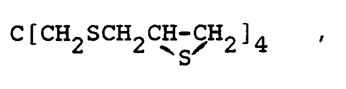
and (2) then reacting this polyepisulfide with hydrogen sulfide to form the first generation polysulfide which can be further reacted with epichlorosulfide and hydrogen sulfide to form subsequent generations. The conditions and procedures which may be suitably employed for polysulfide formation are generally described in Weissberger, Heterocyclic Compounds with Three- and Four-Membered Rings, Interscience Publishers, N.Y., 605 (1964) and Meade et al., J. Chem. Soc., 1894 (1948). Polyaminosulfide dendrimers can be prepared by reacting ammonia or an amine having a plurality of primary amine groups with an excess of ethylene sulfide to form a polysulfide and then with excess aziridine to form a first generation polyaminosulfide which can be reacted with excess ethylene sulfide and then with excess aziridine to form further generations using general reaction conditions described in USP 2,105,845 and Nathan et al., J. Am. Chem. Soc., 63, 2361 (1941). The polyether or polysulfide dendrimers can also be prepared by the excess reactant method by reacting hexahalobenzene with phenol of thiophenol to form a first generation polyarylether or polyarylsulfide and then with excess halogen to form the first generation polyhaloarylpolysulfide and then with further phenol or thiophenol to form further generations according to the procedures and conditions as described by D. D. MacNicol et al., Tetrahedron Letters, 23, 4131-4 (1982).

Illustrative of the partially protected reactant method, a polyol such as pentaerythritol, C(CH₂OH)₄, is employed as the polyvalent core generating compound and is converted to alkali metal salt form, e.g., sodium or lithium, by reaction with alkali metal hydroxide or zero valence alkali metal and then reacted with a molar excess of a partially protected compound such as tosylate ester of 1-ethyl-4-hydroxymethyl-2,6,7-trioxabicyclo[2,2,2]octane to form a protected first generation polyether, 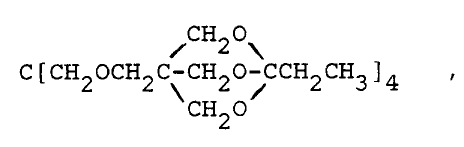
which is then activated by reacting with acid such as hydrochloric acid to form the unprotected first generation polyether, C(CH₂O-CH₂C[CH₂OH]₃)₄. This polyether is converted to alkali metal salt form by reaction with alkali metal hydroxide or zero valence alkali metal and then reacted with a molar excess of the partially protected tosylate ether to form the protected second generation polyether. The foregoing sequence is repeated as desired for additional generation development according to conditions and procedures described in Endo et al., J. Polym. Sci., Polym. Lett. Ed., 18, 457 (1980), Yokoyama et al., Macromolecules, 15, 11-17 (1982), and Pedias et al., Macromolecules, 15, 217-223 (1982). These polyether dendrimers are particularly desirable for use in highly alkaline or highly acidic media wherein hydrolysis of a polyamidoamine dendrimer would be unacceptable. As an example of other dendrimers that are suitably prepared by the partially protected reactant method, polyamine dendrimers may be prepared by reacting ammonia or an amine having a plurality of primary amine groups with N-substituted aziridine such as N-tosyl aziridine, 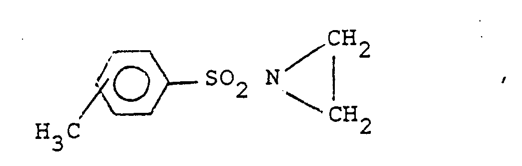
to form a protected first generation polysulfonamide and then activated with acid such as hydrochloric acid to form the first generation polyamine salt and reacted with further N-tosyl aziridine to form the protected second generation polysulfonamide which sequence can be repeated to produce higher generation polyamines using the general reaction conditions described in Humrichause, C. P., PhD Thesis from University of Pennsylvania, "N-Substituted Aziridines as Alkylating Agents", Ref. No. 66-10, 624 (1966).

Examples of other nucleophilic core compounds include phosphine, polyalkylene polyamines such as diethylenetriamine, triethylenetetramine, tetraethylenepentamine and both linear and branched polyethylenimine; primary amines such as hydroxyethylamine, and polymethylenediamines such as hexamethylenediamine; polyaminoalkylarenes such as 1,3,5-tris(aminomethyl)benzene; tris(aminoalkyl)amines such as tris(aminoethy])amine; heterocyclic amines such as imidazolines and piperidines; and various other amines such as hydroxyethylaminoethylamine, mercaptoethylamine, morpholine, piperazine, amino derivatives of polyvinylbenzyl chloride and other benzylic polyamines such as tris(1,3,5-aminomethyl)benzene. Other suitable nucleophilic cores include polyols such as the aforementioned pentaerythritol and polyalkylene polyols and polyalkylene polymercaptans; thiophenols, and phenols. Of the core compounds, ammonia and the polyalkylene polyamines are preferred for the preparation of polyamidoamine dendrimers by the successive excess reactant method and the polyols are preferred for the preparation of polyether dendrimers by the partially protected reactant method.

Examples of coreactant materials used to react with the nucleophilic core compounds include α,β-ethylenically unsaturated carboxylic esters and amides as well as esters, acids and nitriles containing an acrylyl group such as, for example, methyl acrylate, ethyl acrylate, acrylonitrile, methyl itaconate, dimethyl fumarates, maleic annydride, acrylamide, with methyl acrylate being the preferred coreactant material. In general other preferred unsaturated reactants are volatile or otherwise readily removed from the core/coreactant reaction products without deleteriously affecting the reaction product.

Examples of the second coreactant materials used to react with the adduct of the nucleophilic core and the first coreactant include various polyamines such as alkylene polyamines and polyalkylene polyamines such as ethylenediamine and diethylenetriamine; benzylic polyamines such as tris(1,3,5-aminomethyl)benzene; alkanolamines such as ethanolamine; and aziridine and derivatives thereof such as N-aminoethyl aziridine. Of these second coreactant materials, the volatile polyamines such as ethylenediamine and diethylenetriamine are preferred, with ethylenediamine being especially preferred.

Alternatively, the dendrimers can be prepared by reacting an electrophilic core such as a polyester with a coreactant such as a polyamine to form a core adduct which is then reacted with a suitable second coreactant such as an unsaturated ester to form the first generation polyamidoamine. Thereafter, this first generation product is reacted with a suitable third coreactant such as polyamine and then with the second coreactant such as unsaturated ester to form the desired second generation dendrimer. Examples of suitable electrophilic cores include the C₁-C₄ alkyl esters of various polycarboxylic acids such as benzene tricarboxylic acid and various other carboxylic acids represented by the formula: 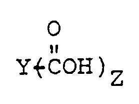
wherein Y is hydrocarbyl or a hydrocarbon polyl wherein the hydrocarbon radical is alkyl, aryl, cycloalkyl, alkylene, arylene, cycloalkylene, and corresponding trivalent, tetravalent, pentavalent and hexavalent radicals of such hydrocarbons; and Z is a whole number from 3 to 6. Preferred electrophilic cores include poly(methyl acrylates), poly(acryloyl chloride), poly(methacryloyl chloride), alkyl acrylate/alkyl methacrylate copolymers, polymers of alkyl fumarates, and polymers of alkyl itaconates. Of the electrophilic cores, alkyl acrylate/alkyl methacrylate copolymers and alkyl acrylate/alkyl itaconate copolymers are most preferred.

Suitable first coreactants for reaction with the electrophilic core include polyalkylene polyamines such as ethylenediamine, diethylenetriamine, triethylenetetramine and other polyamines represented by the formula: 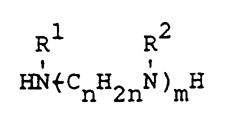
wherein R¹ and R² independently represent hydrogen or an alkyl, preferably C₁-C₄ alkyl, hydroxyalkyl, cyanoalkyl, or amido; n is at least 2 and preferably 2 to 6 and m is 2 to 100, preferably 2 to 5. Examples of suitable second coreactants to be used in preparing dendrimers from electrophilic cores include alkyl esters of ethylenically unsaturated carboxylic acids such as methyl acrylate, methyl methacrylate, ethyl acrylate and the like. Examples of suitable third coreactants are those illustrated for the first coreactant.

Thus prepared, the dendrimers can be reacted with a wide variety of compounds to produce the polyfunctional compounds having the unique characteristics that are attributable to the structure of the dendrimer. For example, a dendrimer having terminal amine groups , as in the polyamidoamine dendrimer, may be reacted with an unsaturated nitrile to yield a polynitrile (nitrile-terminated) dendrimer. Alternatively, the polyamidoamine dendrimer may be reacted with (1) an α,β-ethylenically unsaturated amide to form a polyamide (amide-terminated) dendrimer, (2) an α,β-ethylenically unsaturated ester to form a polyester (ester-terminated) dendrimer, (3) an oxirane to yield a polyol (hydroxy-terminated) dendrimer, or (4) an ethylenically unsaturated sulfide to yield a polymercapto (thiol-terminated) dendrimer. In addition, the dendrimer may be reacted with an appropriate difunctional or trifunctional compound such as an alkyl dihalide or an aromatic diisocyanate to form a poly(dendrimer) having a plurality of dendrimers linked together through the residues of the polyhalide or polyisocyanate. In all instances, such derivatives of the dendrimers are prepared using procedures and conditions conventional for carrying out reactions of organic compounds bearing the particular functional group with the particular organic reactant.

Such reactions are further exemplified by the following working examples. In such working examples, all parts and percentages are by weight unless otherwise indicated.

### Example 1

### A. Preparation of Core Adduct

To a one-liter, 3-neck flask equipped with stirrer, condenser and thermowell, and containing methyl acrylate (296.5 g, 3.45 moles) was added at room temperature with stirring over a 6-hour period ammonia (8.7 g, 0.58 mole) dissolved in 102.2 g of methanol. The mixture was allowed to stand at room temperature for 48 hours at which point excess methyl acrylate was removed by vacuum distillation (1 mm Hg (130 Pa) at 22°C) yielding 156 g of residue. This residue is analyzed by size exclusion chromatography, C₁₃ NMR and liquid chromatography. This analysis indicated the coreactant adduct to be the Michael's addition product of 1 mole of ammonia and 3 moles of methyl acrylate at a 97.8 percent yield.

### B. Preparation of First Generation Adduct

To ethylenediamine (505.8 g, 8.43 moles) dissolved in 215.4 g of methanol in a 3-liter reaction flask equipped with stirrer, condenser and thermowell, was added the aforementioned ammonia/methyl acrylate adduct (28.1 g, 0.1022 mole), and the reaction mixture was allowed to stand at room temperature for 55 hours. The resulting mixture (747.6 g) was subjected to vacuum distillation to remove excess ethylenediamine and methanol at 2 mm Hg (270 Pa) and 72°C. The residue (35.4 g) was analyzed by size exclusion chromatography and other suitable analytical techniques. The analyses indicated that essentially all of the ester moieties of the ammonia/methyl acrylate adduct had been converted to amides in the form of a compound represented by the following structural formula: 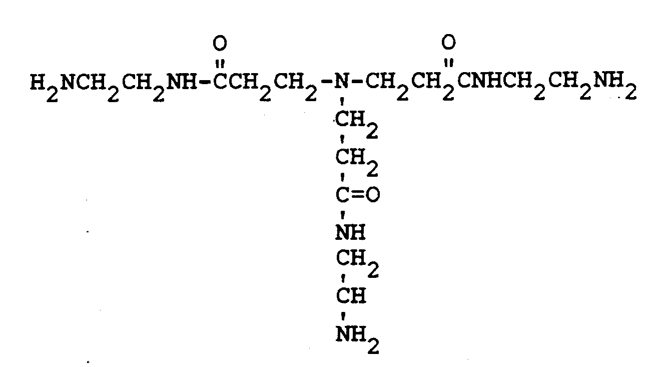
thus indicating a yield of 98.6 percent.

### C. Preparation of Second Generation Polyester Dendrimer

To methyl acrylate (93.2 g, 1.084 moles) in a one-liter flask equipped with condenser, stirrer and thermowell, and heated to 32°C was added the aforementioned first generation adduct (18 g, 0.0501 mole) dissolved in 58.1 g of methanol over 1.5 hours. The resulting mixture was maintained at 32°C for an additional 5 hours and allowed to stand an additional 18 hours at room temperature. The reaction mixture (165.7 g) was stripped of methanol and excess methyl acrylate by vacuum distillation (2 mm Hg (270 Pa) and 50°C) to produce 43.1 g of residue. Analysis by suitable techniques indicated the product to be a second generation polyester dendrimer represented by the following formula: 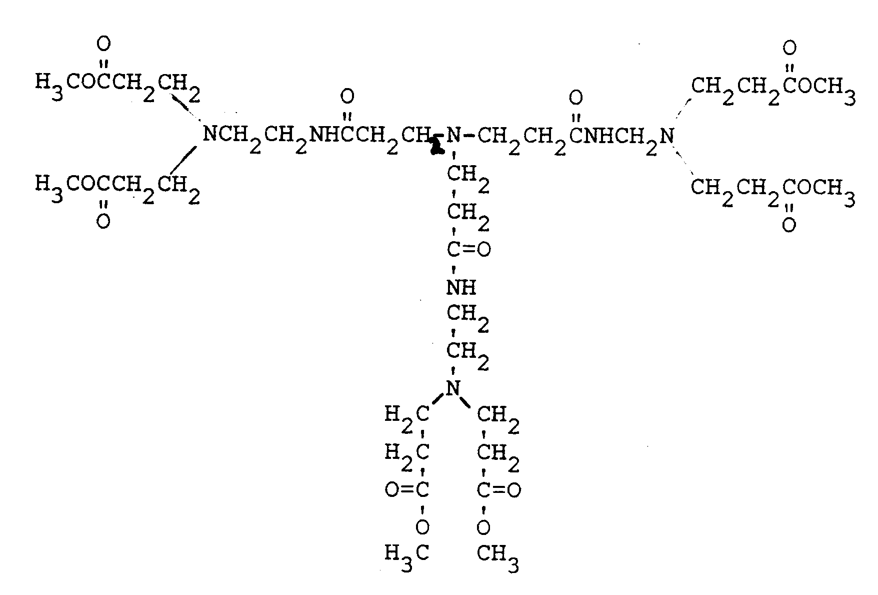
in 98.4 percent yield.

### D. Preparation of Second Generation Polyamine Dendrimer

To ethylenediamine (328.8 g, 5.48 moles) dissolved in 210.2 g of methanol at room temperature in the aforementioned flask was added with stirring the second generation polyester dendrimer (34.9 g, 0.0398 mole) dissolved in 45.3 g of methanol. The resulting mixture was allowed to stand for 66 hours at room temperature at which time excess ethylenediamine and methanol was stripped from the product by vacuum distillation (2 mm Hg (270 Pa) at 72°C) to yield 41.1 g (99.0 percent yield) of product. This product was determined by size exclusion chromatography to be the second generation polyamine of the aforementioned polyester dendrimer.

### E. Preparation of Third Generation Polyester Dendrimer

To methyl acrylate (65.1 g, 0.757 mole) was added the aforementioned second generation polyamine dendrimer (28.4 g, 0.0272 mole) dissolved in 84.6 g of methanol over a period of 1 hour and 15 minutes. The resulting mixture was allowed to stand for 18 hours at 25°C after which time excess methyl acrylate and methanol were removed by vacuum distillation (2 mm Hg (270 Pa) at 50°C) to yield 56.3 g (100.0 percent yield) of product residue. Analysis of this residue by suitable analytical techniques indicated that it was a third generation polyester dendrimer having 3 core branches with 4 terminal ester moieties per core branch thereby providing 12 terminal ester moieties per dendrimer molecule.

### F. Preparation of Third Generation Polyamine Dendrimer

To ethylenediamine (437.6 g, 7.29 moles) dissolved in 192 g of methanol was added the aforementioned third generation polyester dendrimer (44.9 g, 0.0216 mole) dissolved in 69.7 g of methanol. The addition occured over a period of 48 hours at 25°C with stirring. The resulting reaction mixture was then allowed to stand for 19 hours at 25°C after which time excess methanol and ethylenediamine were removed by vacuum distillation (2 mm Hg (270 Pa) at 72°C) to yield 51.2 g of residual product. Analysis of this residue indicated a yield of 85.3 percent of a third generation polyamine dendrimer having 3 core branches with 4 terminal primary amine moieties per core branch, thereby providing 12 terminal primary amine moieties per molecule of dendrimer. This dendrimer was calculated to have a molecular volume between 50,000 and 97,000 cubic Å (50 and 97 nm³) and a density of a terminal amine moiety between 1 to 3(x 10⁻⁴) moieties/cubic Å (0.1 and 0.3 moieties/nm³).

### Example 2

Following the procedure of Example 1, except that a molar equivalent amount of ethylenediamine was substituted for ammonia as the core compound, a third generation polyamine dendrimer is prepared. Upon analysis, it is determined that this dendrimer has four core branches with 4 terminal primary amine moieties per core branch, thereby providing 16 terminal primary amine moieties per molecule of dendrimer. This dendrimer has a molecular volume between 60,000 and 120,000 cubic Å (60 and 120 nm³) and a terminal amine density of 2 to 6(x 10⁻⁴) amines/cubic Å (0.2 and 0.6 amines/nm³).

Similar dendrimers were obtained when equimolar amounts of 1,2-diaminopropane, 1,3-diaminopropane and 1,6-diaminohexane (hexamethylenediamine) were substituted for the ethylenediamine as the core compound in the foregoing procedure. When an equimolar amount of dodecylamine or benzylamine was substituted for the ethylenediamine as the core compound, the resulting dense star polymers had 2 core branches per molecule with 4 terminal primary amine groups per branch, thereby providing a total of 8 primary amine groups per polymer molecule. Substitution of triaminoethylamine for ethylenediamine as the core compound yielded a dendrimer having 6 core branches with 4 terminal primary amine moieties per core branch, thereby providing 24 terminal primary amine moieties per molecule of dendrimer.

### Example 3

### A. First Amidation

A 27.3-g portion (0.1 mole) of a triester represented by the formula: 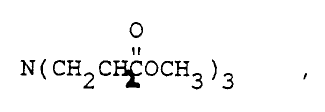
was mixed with 30 g (0.405 mole) of N-methyl ethylenediamine (MEDA) and 16.6 g of methanol and then heated at 63°C for 11 hours. The product was then stripped of unreacted MEDA and methanol to yield 36.1 g of a triamide represented by the formula:

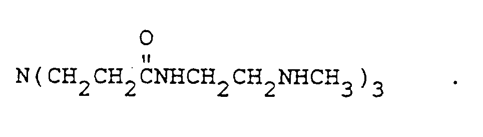

### B. First Alkylation

To the aforementioned triamide (36.1 g, 0.09 mole) was added 38.5 g of methanol to yield a clear solution to which was added 50.5 g (0.59 mole) of methyl acrylate dropwise over a period of 2 hours at 38°C. The temperature of the resulting mixture was increased to 53°C for 5 additional hours after which unreacted methyl acrylate and methanol were removed under vacuum to yield 61 g of a light yellow syrup. Analysis of this product by nuclear magnetic resonance (H¹) spectroscopy indicated that it is represented by the formula:

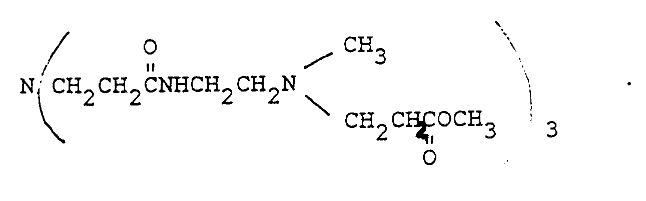

### C. Second Amidation

To 60.8 g of the aforementioned first alkylation product were added with stirring 42.7 g of methanol and 26.6 g (0.359 mole) of MEDA followed by heating the resulting mixture at 65°C for 6 hours. Vacuum stripping of the mixture yielded 72.7 g of a light yellow syrup. Analysis of this product (syrup) indicated that it was a mixture of isomers having the following structures:

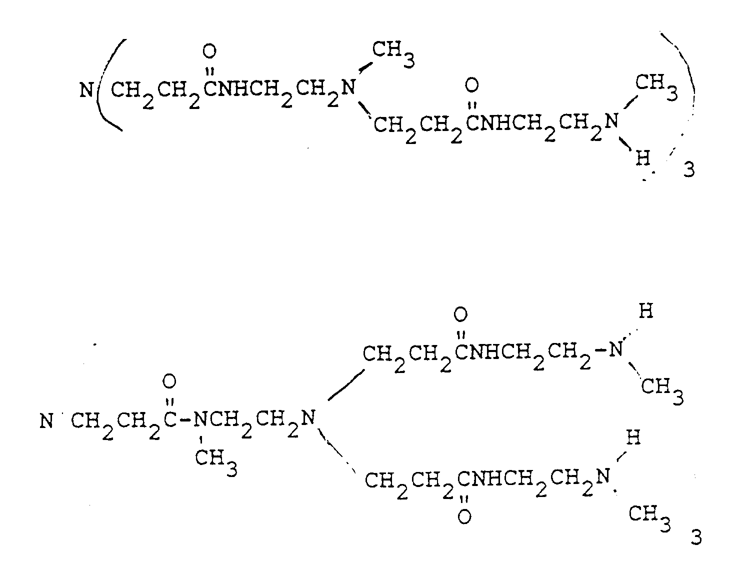

### D. Second Alkylation and Third Amidation

Alkylation of the aforementioned second amidation product with methyl acrylate and then amidation of the resulting alkylated product with MEDA in accordance with aforementioned procedures yielded a mixture of isomers having core branches with dendritic characteristics.

### Example 4 - Demulsification Method

To 100 ml of an oil-in-water emulsion containing about 5 percent of crude oil having a specific gravity of ∼0.98 g/ml was added one part per million based on the emulsion of the dendrimer (ethylene diamine core) of Example 2. The emulsion was then shaken for 3 minutes to effectively disperse the dendrimer into the emulsion. The emulsion was allowed to stand for 10 minutes and visually evaluated. After 10 minutes, the emulsion appeared to be completely resolved into two phases having a distinct interface wherein the aqueous phase was essentially transparent.

Following the foregoing procedure except substituting a quaternized form of the foregoing dendrimer for the dendrimer, the emulsion was similarly resolved using 0.5 ppm and 1 ppm of the quaternized form. This quaternized form was prepared by reacting the 32.42 g (0.01 mole) of the dendrimer in 100 ml of methanol with 24.32 g (0.16 mole) of 2-hydroxy-3-chloropropyl trimethyl ammonium chloride in 30 ml of water at 50°C for 12 hours.

## Claims (Claims for the following Contracting State(s): BE, CH, DE, FR, GB, IT, LI, NL, SE)

1. A dense star polymer having at least three symmetrical core branches emanating from a core, each core branch having at least one terminal group, wherein (1) the ratio of terminal groups to the core branches is greater than 2:1, (2) the density of terminal groups per unit volume in the polymer is at least 1.5 times that of a conventional star polymer having the same core, monomeric components, molecular weight and number of core branches, said monomeric components being such that each of the branches bears only one terminal group, and (3) the molecular volume is not more than 60 percent of the molecular volume of said conventional star polymer.

2. The symmetrical dense star polymer of Claim 1 wherein the core is a nucleophilic atom or group.

3. The symmetrical dense star polymer of Claim 2 wherein the nucleophilic atom or group is phosphine, polyalkylene polyamine, linear or branched polyethylenimine, primary amine, polymethylenediamine, polyaminoalkylarene, tris(aminoalkyl)amine, heterocyclic amine, hydroxyethylaminoethylamine, mercaptoethylamine, morpholine, piperazine, amino derivatives of polyvinylbenzyl chloride, benzylic polyamine, polyol, 1,2-dimercaptoethane, polyalkylene polymercaptans, thiophenols or phenols.

4. The symmetrical dense star polymer of Claim 3 wherein the polyalkylene polyamine is diethylenetriamine, triethylenetetramine or tetraethylenepentamine.

5. The symmetrical dense star polymer of Claim 3 wherein the primary amine is methylamine, hydroxyethylamine or octadecylamine.

6. The symmetrical dense star polymer of Claim 3 wherein the polyol is pentaerythritol, ethylene glycol, polyethylene glycol or polypropylene glycol.

7. The symmetrical dense star polymer of Claim 1 wherein the core is an electrophilic atom or group.

8. The symmetrical dense star polymer of claim 7 wherein the electrophilic atom or group is an adduct of a polyester and polyamine, C₁-C₄ alkyl ester of a polycarboxylic acid, or a carboxylic acid of the formula Y-[C(O)-OH]_{z} where Y is hydrocarbyl or a hydrocarbon polyol where the hydrocarbon radical is alkyl, aryl, cycloalkyl, alkylene, arylene, cycloalkylene or corresponding trivalent, tetravalent, pentavalent or hexavalent radicals of such hydrocarbons; and Z is a whole number from 1 to 6.

9. The symmetrical dense star polymer of Claim 8 wherein the electrophilic atom or group is a poly(methyl acrylate), poly(acryloyl chloride), poly(methacryoyl chloride), alkyl acrylate/alkyl methacrylate copolymer, polymers of alkyl fumarate, polymers of alkyl itaconate or alkyl acrylate/alkyl itaconate copolymer.

10. The symmetrical dense star polymer of Claim 1 wherein the dendritic branches contain amidoamine linkages.

11. The symmetrical dense star polymer of Claim 1 wherein the core is derived from a nucleophilic compound and the core branches are polyamidoamines wherein the terminal groups are primary amine groups.

12. The symmetrical dense star polymer of Claim 1 wherein the terminal group is an amine, carboxylic ester, carboxy, aziridinyl, oxazolinyl, haloalkyl, oxiranyl, hydroxy or isocyanato group.

13. The symmetrical dense star polymer of Claim 1 wherein the core is nucleophilic and derived from a core compound having a plurality of active hydrogens capable of undergoing a Michael's addition reaction with an ethylenically unsaturated group.

14. The symmetrical dense star polymer of Claim 10 wherein the branches are polyamidoamine which is derived from the reaction of an alkyl ester of an α,β-ethylenically unsaturated carboxylic acid or an α,β-ethylenically unsaturated amide and an alkylene polyamine or a polyalkylene polyamine.

15. The symmetrical dense star polymer of Claim 14, wherein the nucleophilic compound is ammonia, the ester is methyl acrylate and the polyamine is ethylenediamine.

16. The symmetrical dense star polymer of Claim 1 wherein the core is derived from ammonia or an amine having a plurality of the primary amine groups and an N-substituted aziridine.

17. The symmetrical dense star polymer of Claim 16 wherein the core is derived from diethylenetriamine and aziridine, and the branches are derived from N-substituted-aziridine.

18. The symmetrical dense star polymer of Claim 17 wherein the N-substituted aziridine is N-tosyl-aziridine.

19. A process for producing a symmetrical dense star polymer of Claim 1 comprising the steps of:
(i) contacting
(1) a core compound having at least three nucleophilic or three electrophilic reactive atoms or groups (A₁-groups) with
(2) an excess of a first organic coreactant having
(a) one core reactive atom or group B₁ which is reactive with the A₁-groups and
(b) at least two reactive atoms or groups A₂ which do not react with the A₁-group or the B₁-group under conditions sufficient to form a core adduct wherein each A₁-group has reacted with the core reactive group of a different molecule of the first coreactant;
(ii) contacting
(1) the core adduct having at least twice the number of reactive groups as the core compound with
(2) an excess of a second organic coreactant having
(a) one adduct reactive atom or group B₂ which will react with the A₂-groups of the core adduct and
(b) at least one reactive atom or group A₃ which does not react with the A₂-groups or the B₂-group under conditions sufficient to form a first generation adduct having a number of reactive groups that are at least twice the number of A₁-groups;
(iii) contacting
the first generation adduct of step (ii) with an excess of a third organic coreactant having
(a) one reactive atom or group B₃ that is reactive with the A₃-groups of the first generation adduct and
(b) at least one reactive atom or group A₄ that does not react with the A₃-groups or the B₃-groups of the first generation adduct under conditions sufficient to form a second generation adduct,
wherein the first coreactant differs from the second coreactant, and the second coreactant differs from the third coreactant, but the first and third coreactants may be the same or different compounds,
and optionally repeating steps (ii) and (iii) the desired number of times and
wherein each excess is 2:1 to 120:1 on a molar basis.

20. The process of Claim 19 wherein
(a) the core compound is ammonia which is contacted with the first organic coreactant, methyl acrylate, to form the core adduct;
(b) the core adduct is then contacted with the second organic coreactant, ethylenediamine, to form the first generation adduct;
(c) the first generation adduct is then reacted with the third organic coreactant, methyl acrylate; and
(d) optionally repeating steps (b) and (c) the desired number of times.

21. A process according to claim 19 comprising the steps of:
(i) contacting
a polyol core with an alkali metal hydoxide or zero valence alkali metal to form the alkali metal salt polyol;
(ii) contacting
the alkali metal salt polyol with a molar excess of a tosylate ester of 1-ethyl-4-hydroxymethyl-2,6,7-trioxabicyclo[2.2.2]octane to form a first generation protected polyether;
(iii) contacting
the protected polyether with acid to form the unprotected first generation polyether;
(iv) optionally repeating steps (ii) and (iii) to form the desired number of generations of the dense star polymer.

22. The process of claim 19 wherein the core compound is reacted with a coreactant selected from the group consisting of ethyl acrylate, acrylonitrile, methyl itaconate, dimethyl fumarates, maleic anhydride or acrylamide.

## Claims (Claims for the following Contracting State(s): AT)

1. A process for producing a symmetrical dense star polymer having at least three symmetrical core branches emanating from a core, each core branch having at least one terminal group, wherein (1) the ratio of terminal groups to the core branches is greater than 2:1, (2) the density of terminal groups per unit volume in the polymer is at least 1.5 times that of a conventional star polymer having the same core, monomeric components, molecular weight and number of core branches, said monomeric components being such that each of the branches bears only one terminal group, and (3) the molecular volume is not more than 60 percent of the molecular volume of said conventional star polymer comprising the steps of:
(i) contacting
(1) a core compound having at least three nucleophilic or three electrophilic reactive atoms or groups (A₁-groups) with
(2) an excess of a first organic coreactant having
(a) one core reactive atom or group B₁ which is reactive with the A₁-groups and
(b) at least two reactive atoms or groups A₂ which do not react with the A₁-group or the B₁-group under conditions sufficient to form a core adduct therein each A₁-group has reacted with the core reactive group of a different molecule of the first coreactant;
(ii) contacting
(1) the core adduct having at least twice the number of reactive groups as the core compound with
(2) an excess of a second organic coreactant having
(a) one adduct reactive atom or group B₂ which will react with the A₂-groups of the core adduct and
(b) at least one reactive atom or group A₃ which does not react with the A₂-groups or the B₂-group under conditions sufficient to form a first generation adduct having a number of reactive groups that are at least twice the number of A₁-groups;
(iii) contacting
the first generation adduct of step (ii) with an excess of a third organic coreactant having
(a) one reactive atom or group B₃ that is reactive with the A₃-groups of the first generation adduct and
(b) at least one reactive atom or group A₄ that does not react with the A₃-groups or the B₃-groups of the first generation adduct under conditions sufficient to form a second generation adduct,
wherein the first coreactant differs from the second coreactant, and the second coreactant differs from the third coreactant, but the first and third coreactants may be the same or different compounds,
and optionally repeating steps (ii) and (iii) the desired number of times and
wherein each excess is 2:1 to 120:1 on a molar basis.

2. The process of claim 1 wherein the nucleophilic atom or group of the core is phosphine, polyalkylene polyamine, linear or branched polyethylenimine, primary amine, polymethylenediamine, polyaminoalkylarene, tris(aminoalkyl)amine, heterocyclic amine, hydroxyethylaminoethylamine, mercaptoethylamine, morpholine, piperazine, amino derivatives of polyvinylbenzyl chloride, benzylic polyamine, polyol, 1,2-dimercaptoethane, polyalkylene polymercaptans, thiophenols or phenols.

3. The process of claim 2 wherein the polyalkylene polyamine is diethylenetriamine, triethylenetetramine or tetraethylenepentamine.

4. The process of claim 2 wherein the primary amine is methylamine, hydroxyethylamine or octadecylamine.

5. The process of claim 2 wherein the polyol is pentaerythritol, ethylene glycol, polyethylene glycol or polypropylene glycol.

6. The process of Claim 1 wherein
(a) the core compound is ammonia which is contacted with the first organic coreactant, methyl acrylate, to form the core adduct;
(b) the core adduct is then contacted with the second organic coreactant, ethylenediamine, to form the first generation adduct;
(c) the first generation adduct is then reacted with the third organic coreactant, methyl acrylate; and
(d) optionally repeating steps (b) and (c) the desired number of times.

7. A process according to claim 2 comprising the steps of:
(i) contacting
a polyol core with an alkali metal hydoxide or zero valence alkali metal to form the alkali metal salt polyol;
(ii) contacting
the alkali metal salt polyol with a molar excess of a tosylate ester of 1-ethyl-4-hydroxymethyl-2,6,7-trioxabicyclo[2.2.2]octane to form a first generation protected polyether;
(iii) contacting
the protected polyether with acid to form the unprotected first generation polyether;
(iv) optionally repeating steps (ii) and (iii) to form the desired number of generations of the dense star polymer.

8. The process of claim 1 wherein the core compound is reacted with a coreactant selected from the group consisting of ethyl acrylate, acrylonitrile, methyl itaconate, dimethyl fumarates, maleic anhydride or acrylamide.

9. The process of claim 1 wherein the electrophilic atom or group of the core is an adduct of a polyester and polyamine, C₁-C₄ alkyl ester of a polycarboxylic acid, or a carboxylic acid of the formula Y-[C(O)-OH]_{z} where Y is hydrocarbyl or a hydrocarbon polyol where the hydrocarbon radical is alkyl, aryl, cycloalkyl, alkylene, arylene, cycloalkylene or corresponding trivalent, tetravalent, pentavalent or hexavalent radicals of such hydrocarbons; and Z is a whole number from 1 to 6.

10. The process of claim 9 wherein the electrophilic atom or group is a poly(methyl acrylate), poly(acryloyl chloride), poly(methacryoyl chloride), alkyl acrylate/alkyl methacrylate copolymer, polymers of alkyl fumarate, polymers of alkyl itaconate or alkyl acrylate/alkyl itaconate copolymer.

11. The process of claim 1 wherein the dendritic branches contain amidoamine linkages.

12. The process of claim 1 wherein the core is derived from a nucleophilic compound and the core branches are polyamidoamines wherein the terminal groups are primary amine groups.

13. The process of claim 1 wherein the terminal group is an amine, carboxylic ester, carboxy, aziridinyl, oxazolinyl, haloalkyl, oxiranyl, hydroxy or isocyanato group.

14. The process of claim 1 wherein the core is nucleophilic and derived from a core compound having a plurality of active hydrogens capable of undergoing a Michael's addition reaction with an ethylenically unsaturated group.

15. The process of claim 11 wherein the branches are polyamidoamine which are prepared by the reaction of an alkyl ester of an α,β-ethylenically unsaturated carboxylic acid or an α,β-ethylenically unsaturated amide and an alkylene polyamine or a polyalkylene polyamine.

16. The process of claim 15 wherein the nucleophilic compound is ammonia, the ester is methyl acrylate and the polyamine is ethylenediamine.

17. The process of claim 1 wherein the core is prepared from ammonia or an amine having a plurality of the primary amine groups and an N-substituted aziridine.

18. The process of claim 17 wherein the core is prepared from diethylenetriamine and aziridine, and the branches are derived from N-substituted-aziridine.

19. The process of claim 18 wherein the N-substituted aziridine is N-tosyl-aziridine.

## Patentansprüche (Patentansprüche für folgende(n) Vertragsstaat(en): BE, CH, DE, FR, GB, IT, LI, NL, SE)

1. Dichtes Sternpolymer mit mindestens drei symmetrischen Stammästen die von einem Kern ausgehen, wobei jeder Stammast mindestens eine endständige Gruppe hat, wobei (1) das Verhältnis endständiger Gruppen zu den Stammästen größer als 2:1 ist, (2) die Dichte endständiger Gruppen pro Volumeneinheit im Polymer mindestens 1.5 Mal der Dichte eines herkömmlichen Sternpolymers mit dem gleichen Kern, den gleichen monomeren Komponenten, dem gleichen Molekulargewicht und der gleichen Zahl von Stammästen beträgt, wobei die monomeren Komponenten von der Art sind, daß jeder der Äste nur eine endständige Gruppe trägt, und (3) das Molekularvolumen nicht größer ist als 60 Prozent des Molekularvolumens des herkömmlichen Sternpolymers.

2. Symmetrisches dichtes Sternpolymer nach Anspruch 1, wobei der Kern ein nukleophiles Atom oder eine nukleophile Gruppe ist.

3. Symmetrisches dichtes Sternpolymer nach Anspruch 2, wobei das nukleophile Atom oder die nukleophile Gruppe Phosphin, Polyalkylenpolyamin, lineares oder verzweigtes Polyethylenimin, primäres Amin, Polymethylendiamin, Polyaminoalkylaren , Tris(aminoalkyl)amin, heterocyclisches Amin, Hydroxyethylaminoethylamin, Mercaptoethylamin, Morpholin, Piperazin, Aminoderivate von Polyvinylbenzylchlorid, Benzylpolyamin, Polyol, 1,2-Dimercaptoethan, Polyalkylenmercaptane, Thiophenole oder Phenole ist.

4. Symmetrisches dichtes Sternpolymer nach Anspruch 3, wobei das Polyalkylenpolyamin Diethylentriamin, Trieethylentetramin oder Tetraethylenpentamin ist.

5. Symmetrisches dichtes Sternpolymer nach Anspruch 3, wobei das primäre Amin Methylamin, Hydroxyethylamin oder Octadecylamin ist.

6. Symmetrisches dichtes Sternpolymer nach Anspruch 3, wobei das Polyol Pentaerythritol, Ethylenglykol, Polyethylenglykol oder Polyproylenglykol ist.

7. Symmetrisches dichtes Sternpolymer nach Anspruch 1, wobei der Kern ein elektrophiles Atom oder eine elektrophile Gruppe ist.

8. Symmetrisches dichtes Sternpolymer nach Anspruch 7, wobei das elektrophile Atom oder die elektrophile Gruppe ein Addukt eines Polyesters und Polyamins, ein C₁-C₄ Alkylester einer Polycarbonsäure, oder eine Carbonsäure der Formel Y-[C(O)-OH]_{z} ist, wobei Y Kohlenwasserstoff oder ein Kohlenwasserstoffpolyol ist, wobei der Kohlenwasserstoffrest Alkyl, Aryl, Cycloalkyl, Alkylen, Arylen, Cycloalkylen, oder entsprechende trivalente, tetravalente, pentavalente oder hexavalente Reste derartiger Kohlenwasserstoffe ist, und Z eine ganze Zahl von 1 bis 6 ist.

9. Symmetrisches dichtes Sternpolymer nach Anspruch 8, wobei das elektrophile Atom oder die elektrophile Gruppe ein Poly(methylacrylat), Poly(acryloylchlorid), Poly(methacryloylchlorid), Alkylacrylat/Alkylmethacrylat Copolymer, Alkylfumaratpolymere, Alkylitaconatpolymere oder ein Alkylacrylat/Alkylitaconat Copolymer ist.

10. Symmetrisches dichtes Sternpolymer nach Anspruch 1, wobei die dendritischen Äste Amidoaminverknüpfungen enthalten.

11. Symmetrisches dichtes Sternpolymer nach Anspruch 1, wobei der Kern von einer nukleophilen Verbindung abstammt und die Stammäste Polyamidoamine sind, wobei die endständigen Gruppen primäre Amingruppen sind.

12. Symmetrisches dichtes Sternpolymer nach Anspruch 1, wobei die endständige Gruppe eine Amin-, Carbonsäureester-, Carboxy-, Aziridinyl-, Oxazolinyl-, Halogenalkyl-, Oxiranyl-, Hydroxy- oder Isocyanatgruppe ist.

13. Symmetrisches dichtes Sternpolymer nach Anspruch 1, wobei der Kern nukleophil ist und von einer Kernverbindung mit mehreren aktiven Wasserstoffen abstammt, die fähig sind mit einer ethylenisch ungesättigten Verbindung eine Michael-Additionsreaktion einzugehen.

14. Symmetrisches dichtes Sternpolymer nach Anspruch 10, wobei die Äste Polyamidoamin sind, das aus der Reaktion eines Alkylesters einer α,β-ethylenisch ungesättigten Carbonsäure oder eines α,β-ethylenisch ungesättigten Amids mit einem Alkylenpolyamin oder einem Polyalkylenpolyamin stammt.

15. Symmetrisches dichtes Sternpolymer nach Anspruch 14, wobei die nukleophile Verbindung Ammoniak ist, der Ester Methylacrylat ist und das Polyamin Ethylendiamin ist.

16. Symmetrisches dichtes Sternpolymer nach Anspruch 1, wobei der Kern von Ammoniak oder einem Amin mit einer Vielzahl der primären Amingruppen und einem N-substituiertem Aziridin abstammt.

17. Symmetrisches dichtes Sternpolymer nach Anspruch 16, wobei der Kern von Diethylentriamin und Aziridin abstammt, und die Äste von N-substituiertem Aziridin abstammen.

18. Symmetrisches dichtes Sternpolymer nach Anspruch 17, wobei das N-substituierte Aziridin N-Tosyl-aziridin ist.

19. Verfahren zum Herstellen eines symmetrischen dichten Sternpolymers nach Anspruch 1, umfassend die Schritte:
(i) in Kontakt Bringen
(1) einer Kernverbindung mit mindestens drei nukleophilen oder drei elektrophilen reaktiven Atomen oder Gruppen (A₁-Gruppen) mit
(2) einem Überschuß eines ersten organischen Co-Reaktanten der aufweist
(a) ein mit dem Kern reaktives Atom oder eine mit dem Kern reaktive Gruppe B₁, das bzw. die mit den A₁-Gruppen reaktiv ist und
(b) mindestens zwei reaktive Atome oder Gruppen A₂, die mit der A₁-Gruppe oder der B₁-Gruppe nicht reagieren unter Bedingungen, die ausreichend sind um ein Kern-Addukt zu bilden, wobei jede A₁-Gruppe mit der mit dem Kern reaktiven Gruppe eines vom ersten Co-Reaktanten verschiedenen Moleküls reagiert hat;
(ii) in Kontakt Bringen
(1) des Kernadduktes, das eine mindestens zweifache Anzahl reaktiver Gruppen als die Kernverbindung hat mit
(2) einem Überschuß eines zweiten organischen Co-Reaktanten der aufweist
(a) ein mit dem Addukt reaktives Atom oder eine mit dem Addukt reaktive Gruppe B₂, das bzw. die mit den A₂-Gruppen des Kernadduktes reagiert und
(b) mindestens ein reaktives Atom oder eine reaktive Gruppe A₃, das bzw. die mit den A₂-Gruppen oder der B₂-Gruppe nicht reagiert unter Bedingungen, die ausreichend sind um ein Addukt der ersten Generation zu bilden, mit einer Anzahl von reaktiven Gruppen, die mindestens das zweifache der Anzahl von A₁-Gruppen beträgt;
(iii) in Kontakt Bringen
des Adduktes der ersten Generation aus Schritt (ii) mit einem Überschuß eines dritten organischen Co-Reaktanten der aufweist
(a) ein reaktives Atom oder eine reaktive Gruppe B₃, das bzw. die mit den A₃-Gruppen des Adduktes der ersten Generation reaktiv ist und
(b) mindestens ein reaktives Atom oder eine reaktive Gruppe A4, das bzw. die mit den A₃-Gruppen oder den B₃-Gruppen des Adduktes der ersten Generation nicht reagiert unter Bedingungen, die ausreichend sind um ein Addukt der zweiten Generation zu bilden,
wobei der erste Co-Reaktant vom zweiten Co-Reaktanten unterschiedlich ist, und der zweite Co-Reaktant vom dritten Co-Reaktanten unterschiedlich ist, aber die ersten und dritten Co-Reaktanten die gleichen oder unterschiedliche Verbindungen sein können,
und gegebenenfalls wiederholen der Schritte (ii) und (iii) so oft wie gewünscht, und
wobei ein Überschuß jeweils von 2:1 bis 120:1 auf molarer Basis beträgt.

20. Verfahren nach Anspruch 19, wobei
(a) die Kernverbindung Ammoniak ist, der mit dem ersten organischen Co-Reaktanten, Methylacrylat, in Kontakt gebracht wird um das Kernaddukt zu bilden,
(b) das Kernaddukt dann mit dem zweiten organischen Co-Reaktanten, Ethylendiamin, in Kontakt gebracht wird um das Addukt der ersten Generation zu bilden,
(c) das Addukt der ersten Generation dann mit dem dritten organischen Co-Reaktanten, Methylacrylat, in Kontakt gebracht wird, und
(d) gegebenenfalls Wiederholen der Schritte (b) und (c) so oft wie gewünscht.

21. Verfahren nach Anspruch 19, umfassend die Schritte
(i) in Kontakt Bringen
eines Polyolkerns mit einem Alkalimetallhydroxid oder mit einem Alkalimetall der Oxidationsstufe null, um das Alkalimetallsalz des Polyols zu bilden,
(ii) in Kontakt Bringen
das Alkalimetallsalz des Polyols mit einem molaren Überschuß eines Tosylatesters von 1-Ethyl-4-hydroxymethyl-2,6,7-trioxabicyclo[2.2.2.]octan, um einen geschützten Polyether der ersten Generation zu bilden,
(iii) in Kontakt Bringen
des geschützten Polyethers mit Säure , um den ungeschützten Polyether der ersten Generation zu bilden,
(iv) gegebenenfalls Wiederholen der Schritte (ii) und (iii) um die gewünschte Generationszahl des dichten Sternpolymers zu bilden.

22. Verfahren nach Anspruch 19, wobei die Kernverbindung mit einem Co-Reaktanten umgesetzt wird ausgewählt aus der Gruppe bestehend aus Ethylacrylat, Acrylnitril, Methylitaconat, Dimethylfumaraten, Maleinsäureanhydrid oder Acrylamid.

## Patentansprüche (Patentansprüche für folgende(n) Vertragsstaat(en): AT)

1. Verfahren zum Herstellen eines symmetrischen dichten Sternpolymers mit mindestens drei symmetrischen Stammästen die von einem Kern ausgehen, wobei jeder Stammast mindestens eine endständige Gruppe hat, wobei (1) das Verhältnis endständiger Gruppen zu den Stammästen größer als 2:1 ist, (2) die Dichte endständiger Gruppen pro Volumeneinheit im Polymer mindestens 1.5 Mal der Dichte eines herkömmlichen Sternpolymers mit dem gleichen Kern, den gleichen monomeren Komponenten, dem gleichen Molekulargewicht und der gleichen Zahl von Stammästen beträgt, wobei die monomeren Komponenten von der Art sind, daß jeder der Äste nur eine endständige Gruppe trägt, und (3) das Molekularvolumen nicht größer ist als 60 Prozent des Molekularvolumens des herkömmlichen Sternpolymers, umfassend die Schritte:
(i) in Kontakt Bringen
(1) einer Kernverbindung mit mindestens drei nukleophilen oder drei elektrophilen reaktiven Atomen oder Gruppen (A₁-Gruppen) mit
(2) einem Überschuß eines ersten organischen Co-Reaktanten der aufweist
(a) ein mit dem Kern reaktives Atom oder eine mit dem Kern reaktive Gruppe B₁, das bzw. die mit den A₁-Gruppen reaktiv ist und
(b) mindestens zwei reaktive Atome oder Gruppen A₂, die mit der A₁-Gruppe oder der B₁-Gruppe nicht reagieren unter Bedingungen, die ausreichend sind um ein Kern-Addukt zu bilden, wobei jede A₁-Gruppe mit der mit dem Kern reaktiven Gruppe eines vom ersten Co-Reaktanten verschiedenen Moleküls reagiert hat;
(ii) in Kontakt Bringen
(1) des Kernadduktes, das eine mindestens zweifache Anzahl reaktiver Gruppen als die Kernverbindung hat mit
(2) einem Überschuß eines zweiten organischen Co-Reaktanten der aufweist
(a) ein mit dem Addukt reaktives Atom oder eine mit dem Addukt reaktive Gruppe B₂, das bzw. die mit den A₂-Gruppen des Kernadduktes reagiert und
(b) mindestens ein reaktives Atom oder eine reaktive Gruppe A₃, das bzw. die mit den A₂-Gruppen oder der B₂-Gruppe nicht reagiert unter Bedingungen, die ausreichend sind um ein Addukt der ersten Generation zu bilden, mit einer Anzahl von reaktiven Gruppen, die mindestens das zweifache der Anzahl von A₁-Gruppen beträgt;
(iii) in Kontakt Bringen des Adduktes der ersten Generation aus Schritt (ii) mit einem Überschuß eines dritten organischen Co-Reaktanten der aufweist
(a) ein reaktives Atom oder eine reaktive Gruppe B₃, das bzw. die mit den A₃-Gruppen des Adduktes der ersten Generation reaktiv ist und
(b) mindestens ein reaktives Atom oder eine reaktive Gruppe A4, das bzw. die mit den A₃-Gruppen oder den B₃-Gruppen des Adduktes der ersten Generation nicht reagiert unter Bedingungen, die ausreichend sind um ein Addukt der zweiten Generation zu bilden,
wobei der erste Co-Reaktant vom zweiten Co-Reaktanten unterschiedlich ist, und der zweite Co-Reaktant vom dritten Co-Reaktanten unterschiedlich ist, aber die ersten und dritten Co-Reaktanten die gleichen oder unterschiedliche Verbindungen sein können,
und gegebenenfalls Wiederholen der Schritte (ii) und (iii) so oft wie gewünscht, und
wobei ein Überschuß jeweils von 2:1 bis 120:1 auf molarer Basis beträgt.

2. Verfahren nach Anspruch 1, wobei das nukleophile Atom oder die nukleophile Gruppe Phosphin, Polyalkylenpolyamin, lineares oder verzweigtes Polyethylenimin, primäres Amin, Polymethylendiamin, Polyaminoalkylaren , Tris(aminoalkyl)amin, heterocyclisches Amin, Hydroxyethylaminoethylamin, Mercaptoethylamin, Morpholin, Piperazin, Aminoderivate von Polyvinylbenzylchlorid, Benzylpolyamin, Polyol, 1,2-Dimercaptoethan, Polyalkylenmercaptane, Thiophenole oder Phenole ist.

3. Verfahren nach Anspruch 2, wobei das Polyalkylenpolyamin Diethylentriamin, Trieethylentetramin oder Tetraethylenpentamin ist.

4. Verfahren nach Anspruch 2, wobei das primäre Amin Methylamin, Hydroxyethylamin oder Octadecylamin ist.

5. Verfahren nach Anspruch 2, wobei das Polyol Pentaerythritol, Ethylenglykol, Polyethylenglykol oder Polyproylenglykol ist.

6. Verfahren nach Anspruch 1, wobei
(a) die Kernverbindung Ammoniak ist, der mit dem ersten organischen Co-Reaktanten, Methylacrylat, in Kontakt gebracht wird um das Kernaddukt zu bilden,
(b) das Kernaddukt dann mit dem zweiten organischen Co-Reaktanten, Ethylendiamin, in Kontakt gebracht wird um das Addukt der ersten Generation zu bilden,
(c) das Addukt der ersten Generation dann mit dem dritten organischen Co-Reaktanten, Methylacrylat, in Kontakt gebracht wird, und
(d) gegebenenfalls Wiederholen der Schritte (b) und (c) so oft wie gewünscht.

7. Verfahren nach Anspruch 2, umfassend die Schritte
(i) in Kontakt Bringen
eines Polyolkerns mit einem Alkalimetallhydroxid oder mit einem Alkalimetall der Oxidationsstufe null, um das Alkalimetallsalz des Polyols zu bilden,
(ii) in Kontakt Bringen
das Alkalimetallsalz des Polyols mit einem molaren Überschuß eines Tosylatesters von 1-Ethyl-4-hydroxymethyl-2,6,7-trioxabicyclo[2.2.2.]octan, um einen geschützten Polyether der ersten Generation zu bilden,
(iii) in Kontakt Bringen
des geschützten Polyethers mit Säure , um den ungeschützten Polyether der ersten Generation zu bilden,
(iv) gegebenenfalls Wiederholen der Schritte (ii) und (iii) um die gewünschte Generationszahl des dichten Sternpolymers zu bilden.

8. Verfahren nach Anspruch 1, wobei die Kernverbindung mit einem Co-Reaktanten umgesetzt wird ausgewählt aus der Gruppe bestehend aus Ethylacrylat, Acrylnitril, Methylitaconat, Dimethylfumaraten, Maleinsäureanhydrid oder Acrylamid.

9. Verfahren nach Anspruch 1, wobei das elektrophile Atom oder die elektrophile Gruppe des Kerns ein Addukt eines Polyesters und Polyamins, ein C₁-C₄ Alkylester einer Polycarbonsäure, oder eine Carbonsäure der Formel Y-[C(O)-OH]_{z} ist, wobei Y Kohlenwasserstoff oder ein Kohlenwasserstoffpolyol ist, wobei der Kohlenwasserstoffrest Alkyl, Aryl, Cycloalkyl, Alkylen, Arylen, Cycloalkylen, oder entsprechende trivalente, tetravalente, pentavalente oder hexavalente Reste derartiger Kohlenwasserstoffe ist, und Z eine ganze Zahl von 1 bis 6 ist.

10. Verfahren nach Anspruch 9, wobei das elektrophile Atom oder die elektrophile Gruppe ein Poly(methylacrylat), Poly(acryloylchlorid), Poly(methacryloylchlorid), Alkylacrylat/Alkylmethacrylat Copolymer, Alkylfumaratpolymere, Alkylitaconatpolymere oder ein Alkylacrylat/Alkylitaconat Copolymer ist.

11. Verfahren nach Anspruch 1, wobei die dendritischen Äste Amidoaminverknüpfungen enthalten.

12. Verfahren nach Anspruch 1, wobei der Kern von einer nukleophilen Verbindung abstammt und die Stammäste Polyamidoamine sind, wobei die endständigen Gruppen primäre Amingruppen sind.

13. Verfahren nach Anspruch 1, wobei die endständige Gruppe eine Amin-, Carbonsäureester-, Carboxy-, Aziridinyl-, Oxazolinyl-, Halogenalkyl-, Oxiranyl-, Hydroxy- oder Isocyanatgruppe ist.

14. Verfahren nach Anspruch 1, wobei der Kern nukleophil ist und von einer Kernverbindung mit mehreren aktiven Wasserstoffen abstammt, die fähig ist mit einer ethylenisch ungesättigten Verbindung eine Michael-Additionsreaktion einzugehen.

15. Verfahren nach Anspruch 11, wobei die Äste Polyamidoamin sind, die durch die Reaktion eines Alkylesters einer α,β-ethylenisch ungesättigten Carbonsäure oder eines α,β-ethylenisch ungesättigten Amids mit einem Alkylenpolyamin oder einem Polyalkylenpolyamin hergestellt werden.

16. Verfahren nach Anspruch 15, wobei die nukleophile Verbindung Ammoniak ist, der Ester Methylacrylat ist und das Polyamin Ethylendiamin ist.

17. Verfahren nach Anspruch 1, wobei der Kern aus Ammoniak oder einem Amin mit einer Vielzahl der primären Amingruppen und einem N-substituiertem Aziridin hergestellt wird.

18. Verfahren nach Anspruch 17, wobei der Kern aus Diethylentriamin und Aziridin hergestellt wird, und die Äste von N-substituiertem Aziridin abstammen.

19. Verfahren nach Anspruch 18, wobei das N-substituierte Aziridin N-Tosyl-aziridin ist.

## Revendications (Revendications pour l'(les) Etat(s) contractant(s) suivant(s): BE, CH, DE, FR, GB, IT, LI, NL, SE)

1. Polymère étoilé dense, comportant au moins trois branches centrales symétriques partant d'un noyau central, chaque branche centrale portant au moins un groupe terminal, dans lequel :
1) le rapport du nombre de groupes terminaux au nombre de branches centrales est supérieur à 2/1,
2) la densité de groupes terminaux par unité de volume du polymère vaut au moins 1,5 fois celle d'un polymère étoilé classique ayant le même noyau central, les mêmes composants monomères, la même masse moléculaire et le même nombre de branches centrales, lesdits composants monomères étant tels que chacune des branches ne porte qu'un seul groupe terminal, et
3) le volume moléculaire ne vaut pas plus de 60 % du volume moléculaire dudit polymère étoilé classique.

2. Polymère étoilé dense symétrique conforme à la revendication 1, dans lequel le noyau central est constitué par un atome ou un groupe nucléophile.

3. Polymère étoilé dense symétrique conforme à la revendication 2, dans lequel l'atome ou le groupe nucléophile est constitué par une phosphine, une polyalkylène-polyamine, une polyéthylène-imine linéaire ou ramifiée, une amine primaire, une polyméthylène-diamine, un polyaminoalkyl-arène, une tris(aminoalkyl)amine, une amine hétérocyclique, l'hydroxyéthylaminoéthylamine, la mercaptoéthylamine, la morpholine, la pipérazine, un dérivé aminé de poly(chlorure de vinylbenzyle), une polyamine benzylique, un polyol, le 1,2-éthanedithiol, un polyalkyléne-polythiol, un thiophénol ou un phénol.

4. Polymère étoilé dense symétrique conforme à la revendication 3, dans lequel la polyalkylène-polyamine est la diéthylène-triamine, la triéthylène-tétramine ou la tétraéthylène-pentamine.

5. Polymère étoilé dense symétrique conforme à la revendication 3, dans lequel l'amine primaire est la méthylamine, l'hydroxyéthylamine ou l'octadécylamine.

6. Polymère étoilé dense symétrique conforme à la revendication 3, dans lequel le polyol est le pentaérythritol, l'éthylèneglycol, un polyéthylèneglycol ou un polypropylèneglycol.

7. Polymère étoilé dense symétrique conforme à la revendication 1, dans lequel le noyau central est constitué par un atome ou un groupe électrophile.

8. Polymère étoilé dense symétrique conforme à la revendication 7, dans lequel l'atome ou le groupe électrophile est constitué par un produit d'addition d'un polyester et d'une polyamine, un ester d'alkyle en C₁₋₄ d'un poly(acide carboxylique), ou un acide carboxylique de formule Y(COOH)_{z} où Y représente un groupe hydrocarboné ou polyhydroxy-hydrocarboné dans lequel le groupe hydrocarboné est un groupe alkyle, aryle, cycloalkyle, alkylène, arylène ou cycloalkylène, ou un radical trivalent, tétravalent, pentavalent ou hexavalent correspondant à un tel groupe hydrocarboné, et z représente un nombre entier valant de 1 à 6.

9. Polymère étoilé dense symétrique conforme à la revendication 8, dans lequel l'atome ou le groupe électrophile est constitué par un poly(méthacrylate de méthyle), un poly(chlorure d'acryloyle), un poly(chlorure de méthacryloyle), un copolymère de méthacrylate d'alkyle et d'acrylate d'alkyle, un polymère de fumarate d'alkyle, un polymère d'itaconate d'alkyle ou un copolymère d'acrylate d'alkyle et d'itaconate d'alkyle.

10. Polymère étoilé dense symétrique conforme à la revendication 1, dans lequel les branches dendritiques comportent des chaînons de type amidoamine.

11. Polymère étoilé dense symétrique conforme à la revendication 1, dans lequel le noyau central dérive d'un composé nucléophile et les branches centrales sont des polyamidoamines dont les groupes terminaux sont des groupes amino primaires.

12. Polymère étoilé dense symétrique conforme à la revendication 1, dans lequel les groupes terminaux sont des groupes amino, ester carboxylique, carboxy, aziridinyle, oxazolinyle, halogénoalkyle, oxiranyle, hydroxy ou isocyanato.

13. Polymère étoilé dense symétrique conforme à la revendication 1, dans lequel le noyau central est nucléophile et dérive d'un composé de noyau central et comportant plusieurs atomes d'hydrogène actifs, capables de donner lieu à une réaction d'addition de Michael avec un groupe à insaturation éthylénique.

14. Polymère étoilé dense symétrique conforme à la revendication 10, dans lequel les branches sont des polyamidoamines qui dérivent de la réaction d'un ester alkylique d'un acide carboxylique à insaturation α,β-éthylénique ou d'un amide à insaturation α,β-éthylénique et d'une alkylène-polyamine ou d'une polyalkylène-polyamine.

15. Polymère étoilé dense symétrique conforme à la revendication 14, dans lequel le composé nucléophile est l'ammoniac, l'ester est l'acrylate de méthyle, et la polyamine est l'éthylènediamine.

16. Polymère étoilé dense symétrique conforme à la revendication 1, dans lequel le noyau central dérive de l'ammoniac ou d'une amine comportant plusieurs groupes amino primaires et d'une aziridine N-substituée.

17. Polymère étoilé dense symétrique conforme à la revendication 16, dans lequel le noyau central dérive de la diéthylènetriamine et de l'aziridine, et les branches dérivent d'une aziridine N-substituée.

18. Polymère étoilé dense symétrique conforme à la revendication 17, dans lequel l'aziridine N-substituée est la N-tosyl-aziridine.

19. Procédé de préparation d'un polymère étoilé dense symétrique conforme à la revendication 1, qui comporte les étapes consistant à :
I) mettre
1) un composé de noyau central, comportant au moins trois atomes ou groupes réactifs nucléophiles ou trois atomes ou groupes réactifs électrophiles (groupes A₁), en contact avec
2) un premier coréactif organique en excès, comportant
a) un seul atome ou groupe B₁ réactif vis-à-vis du noyau central, qui réagit avec les groupes A₁, et
b) au moins deux atomes ou groupes réactifs A₂ qui ne réagissent pas avec les groupes A₁ ou B₁,
dans des conditions suffisantes pour former un produit d'addition constituant un noyau central, dans lequel chacun des groupes A₁ a réagi avec le groupe B₁ d'une molécule différente du premier coréactif ;
II) mettre
1) le produit d'addition constituant le noyau central, qui comporte au moins deux fois plus de groupes réactifs que le composé de noyau central, en contact avec
2) un deuxième coréactif organique en excès, comportant
a) un seul atome ou groupe B₂ réactif vis-à-vis du produit d'addition, qui réagit avec les groupes A₂ du produit d'addition constituant le noyau central , et
b) au moins un atome ou groupe réactif A₃, qui ne réagit pas avec les groupes A₂ ou B₂.
dans des conditions suffisantes pour former un produit d'addition de première génération dans lequel le nombre de groupes réactifs vaut au moins deux fois le nombre de groupes A₁ ;
III) mettre le produit d'addition de première génération, issu de l'étape (II), en contact avec un troisième coréactif organique en excès, comportant
a) un seul atome ou groupe réactif B₃, qui réagit avec les groupes A₃ du produit d'addition de première génération, et
b) au moins un atome ou groupe réactif A₄, qui ne réagit pas avec les groupes A₃ ou B₃ du produit d'addition de première génération,
dans des conditions suffisantes pour former un produit d'addition de deuxième génération,
le premier coréactif étant différent du deuxième coréactif et le deuxième coréactif étant différent du troisième coréactif, mais le premier et le troisième coréactifs pouvant être des composés identiques ou différents,
et la répétition éventuelle des étapes (II) et (III), autant de fois qu'on le souhaite,
chacun desdits excès correspondant à un rapport molaire de 2/1 à 120/1.

20. Procédé conforme à la revendication 19, dans lequel
a) le composé de noyau central est de l'ammoniac, que l'on met en contact avec de l'acrylate de méthyle, en tant que premier coréactif organique, pour former le produit d'addition constituant le noyau central ;
b) on met ensuite ce produit d'addition constituant le noyau central en contact avec de l'éthylènediamine, en tant que deuxième coréactif organique, pour former le produit d'addition de première génération ;
c) on fait ensuite réagir ce produit d'addition de première génération avec de l'acrylate de méthyle, en tant que troisième coréactif organique, et
d) on répète éventuellement les étapes (b) et (c), autant de fois qu'on le souhaite.

21. Procédé conforme à la revendication 19, qui comporte les étapes consistant à :
I) mettre un composé de noyau central de type polyol en contact avec un hydroxyde de métal alcalin ou un métal alcalin à l'état de valence nulle, pour former un sel de polyol et de métal alcalin ;
II) mettre ce sel de polyol et de métal alcalin en contact avec de l'ester tosylate de 1-éthyl-4-hydroxyméthyl-2,6,7-trioxabicyclo[2.2.2]octane en excès molaire, pour former un polyéther de première génération protégé ;
III) mettre ce polyéther protégé en contact avec un acide, pour former le polyéther de première génération déprotégé ; et
IV) répéter éventuellement les étapes (II) et (III) pour former le nombre voulu de générations d'un polymère étoilé dense.

22. Procédé conforme à la revendication 19, dans lequel on fait réagir le composé de noyau central avec un coréactif choisi dans l'ensemble que constituent l'acrylate d'éthyle, l'acrylonitrile, l'itaconate de méthyle, le fumarate de diméthyle, l'anhydride maléique et l'acrylamide.

## Revendications (Revendications pour l'(les) Etat(s) contractant(s) suivant(s): AT)

1. Procédé de préparation d'un polymère étoilé dense, comportant au moins trois branches centrales symétriques partant d'un noyau central, chaque branche centrale portant au moins un groupe terminal, dans lequel :
1) le rapport du nombre de groupes terminaux au nombre de branches centrales est supérieur à 2/1,
2) la densité de groupes terminaux par unité de volume du polymère vaut au moins 1,5 fois celle d'un polymère étoilé classique ayant le même noyau central, les mêmes composants monomères, la même masse moléculaire et le même nombre de branches centrales, lesdits composants monomères étant tels que chacune des branches ne porte qu'un seul groupe terminal, et
3) le volume moléculaire ne vaut pas plus de 60 % du volume moléculaire dudit polymère étoilé classique,
ledit procédé comportant les étapes consistant à :
I) mettre
1) un composé de noyau central, comportant au moins trois atomes ou groupes réactifs nucléophiles ou trois atomes ou groupes réactifs électrophiles (groupes A₁), en contact avec
2) un premier coréactif organique en excès, comportant
a) un seul atome ou groupe B₁ réactif vis-à-vis du noyau central, qui réagit avec les groupes A₁, et
b) au moins deux atomes ou groupes réactifs A₂ qui ne réagissent pas avec les groupes A₁ ou B₁,
dans des conditions suffisantes pour former un produit d'addition constituant un noyau central, dans lequel chacun des groupes A₁ a réagi avec le groupe B₁ d'une molécule différente du premier coréactif ;
II) mettre
1) le produit d'addition constituant le noyau central, qui comporte au moins deux fois plus de groupes réactifs que le composé de noyau central, en contact avec
2) un deuxième coréactif organique en excès, comportant
a) un seul atome ou groupe B₂ réactif vis-à-vis du produit d'addition, qui réagit avec les groupes A₂ du produit
d'addition constituant le noyau central , et
b) au moins un atome ou groupe réactif A₃, qui ne réagit pas avec les groupes A₂ ou B₂,
dans des conditions suffisantes pour former un produit d'addition de première génération dans lequel le nombre de groupes réactifs vaut au moins deux fois le nombre de groupes A₁ ;
III) mettre le produit d'addition de première génération, issu de l'étape (II), en contact avec un troisième coréactif organique en excès, comportant
a) un seul atome ou groupe réactif B₃, qui réagit avec les groupes A₃ du produit d'addition de première génération, et
b) au moins un atome ou groupe réactif A₄, qui ne réagit pas avec les groupes A₃ ou B₃ du produit d'addition de première génération,
dans des conditions suffisantes pour former un produit d'addition de deuxième génération,
le premier coréactif étant différent du deuxième coréactif et le deuxième coréactif étant différent du troisième coréactif, mais le premier et le troisième coréactifs pouvant être des composés identiques ou différents,
et la répétition éventuelle des étapes (II) et (III), autant de fois qu'on le souhaite,
chacun desdits excès correspondant à un rapport molaire de 2/1 à 120/1.

2. Procédé conforme à la revendication 1, dans lequel l'atome ou le groupe nucléophile du noyau central est constitué par une phosphine, une polyalkylène-polyamine, une polyéthylène-imine linéaire ou ramifiée, une amine primaire, une polyméthylène-diamine, un polyaminoalkyl-arène, une tris(aminoalkyl)amine, une amine hétérocyclique, l'hydroxyéthylaminoéthylamine, la mercaptoéthylamine, la morpholine, la pipérazine, un dérivé aminé de poly(chlorure de vinylbenzyle), une polyamine benzylique, un polyol, le 1,2-éthanedithiol, un polyalkyléne-polythiol, un thiophénol ou un phénol.

3. Procédé conforme à la revendication 2, dans lequel la polyalkylène-polyamine est la diéthylène-triamine, la triéthylène-tétramine ou la tétraéthylène-pentamine.

4. Procédé conforme à la revendication 2, dans lequel l'amine primaire est la méthylamine, l'hydroxy-éthylamine ou l'octadécylamine.

5. Procédé conforme à la revendication 2, dans lequel le polyol est le pentaérythritol, l'éthylèneglycol, un polyéthylèneglycol ou un polypropylèneglycol.

6. Procédé conforme à la revendication 1, dans lequel
a) le composé de noyau central est de l'ammoniac, que l'on met en contact avec de l'acrylate de méthyle, en tant que premier coréactif organique, pour former le produit d'addition constituant le noyau central ;
b) on met ensuite ce produit d'addition constituant le noyau central en contact avec de l'éthylènediamine, en tant que deuxième coréactif organique, pour former le produit d'addition de première génération ;
c) on fait ensuite réagir ce produit d'addition de première génération avec de l'acrylate de méthyle, en tant que troisième coréactif organique, et
d) on répète éventuellement les étapes (b) et (c), autant de fois qu'on le souhaite.

7. Procédé conforme à la revendication 2, qui comporte les étapes consistant à :
I) mettre un composé de noyau central de type polyol en contact avec un hydroxyde de métal alcalin ou un métal alcalin à l'état de valence nulle, pour former un sel de polyol et de métal alcalin ;
II) mettre ce sel de polyol et de métal alcalin en contact avec de l'ester tosylate de 1-éthyl-4-hydroxyméthyl-2,6,7-trioxabicyclo[2.2.2]octane en excès molaire, pour former un polyéther de première génération protégé ;
III) mettre ce polyéther protégé en contact avec un acide, pour former le polyéther de première génération déprotégé ; et
IV) répéter éventuellement les étapes (II) et (III) pour former le nombre voulu de générations d'un polymère étoilé dense.

8. Procédé conforme à la revendication 1, dans lequel on fait réagir le composé de noyau central avec un coréactif choisi dans l'ensemble que constituent l'acrylate d'éthyle, l'acrylonitrile, l'itaconate de méthyle, le fumarate de diméthyle, l'anhydride maléique et l'acrylamide.

9. Procédé conforme à la revendication 1, dans lequel l'atome ou le groupe électrophile du noyau central est constitué par un produit d'addition d'un polyester et d'une polyamine, un ester d'alkyle en C₁₋₄ d'un poly(acide carboxylique), ou un acide carboxylique de formule Y(COOH)_{z} où Y représente un groupe hydrocarboné ou polyhydroxyhydrocarboné dans lequel le groupe hydrocarboné est un groupe alkyle, aryle, cycloalkyle, alkylène, arylène ou cycloalkylène, ou un radical trivalent, tétravalent, pentavalent ou hexavalent correspondant à un tel groupe hydrocarboné, et z représente un nombre entier valant de 1 à 6.

10. Procédé conforme à la revendication 9, dans lequel l'atome ou le groupe électrophile est constitué par un poly(méthacrylate de méthyle), un poly(chlorure d'acryloyle), un poly(chlorure de méthacryloyle), un copolymère de méthacrylate d'alkyle et d'acrylate d'alkyle, un polymère de fumarate d'alkyle, un polymère d'itaconate d'alkyle ou un copolymère d'acrylate d'alkyle et d'itaconate d'alkyle.

11. Procédé conforme à la revendication 1, dans lequel les branches dendritiques comportent des chaînons de type amidoamine.

12. Procédé conforme à la revendication 1, dans lequel le noyau central dérive d'un composé nucléophile et les branches centrales sont des polyamidoamines dont les groupes terminaux sont des groupes amino primaires.

13. Procédé conforme à la revendication 1, dans lequel les groupes terminaux sont des groupes amino, ester carboxylique, carboxy, aziridinyle, oxazolinyle, halogénoalkyle, oxiranyle, hydroxy ou isocyanato.

14. Procédé conforme à la revendication 1, dans lequel le noyau central est nucléophile et dérive d'un composé de noyau central et comportant plusieurs atomes d'hydrogène actifs, capables de donner lieu à une réaction d'addition de Michael avec un groupe à insaturation éthylénique.

15. Procédé conforme à la revendication 11, dans lequel les branches sont des polyamidoamines qui dérivent de la réaction d'un ester alkylique d'un acide carboxylique à insaturation α,β-éthylénique ou d'un amide à insaturation α,β-éthylénique et d'une alkylène-polyamine ou d'une polyalkylène-polyamine.

16. Procédé conforme à la revendication 15, dans lequel le composé nucléophile est l'ammoniac, l'ester est l'acrylate de méthyle, et la polyamine est l'éthylènediamine.

17. Procédé conforme à la revendication 1, dans lequel le noyau central dérive de l'ammoniac ou d'une amine comportant plusieurs groupes amino primaires et d'une aziridine N-substituée.

18. Procédé conforme à la revendication 17, dans lequel le noyau central dérive de la diéthylènetriamine et de l'aziridine, et les branches dérivent d'une aziridine N-substituée.

19. Procédé conforme à la revendication 18, dans lequel l'aziridine N-substituée est la N-tosyl-aziridine.
